# EUROPÄISCHE PATENTSCHRIFT

(11) **EP 1 908 451 B1**
(45) Veröffentlichungstag und Bekanntmachung des Hinweises auf die Patenterteilung: **04.06.2014**
(21) Anmeldenummer: 07019242.2
(22) Anmeldetag: 01.10.2007
(51) Int. Cl.: A61K 8/06, A61K 8/41, A61K 8/92, A61Q 19/00

(54) **Kosmetische Zubereitungen mit naphthenarmen Mineralölen**
Cosmetic preparations with mineral oils low in naphthene
Préparations cosmétiques à l'aide d'huiles minérales pauvres en naphte

(30) Priorität: 07.10.2006 DE 102006047507
(43) Veröffentlichungstag der Anmeldung: 09.04.2008
(73) Patentinhaber: Beiersdorf AG, 20253 Hamburg (DE)
(72) Erfinder: Nielsen, Jens, 24558 Henstedt-Ulzburg (DE); Riedel, Heidi, 22083 Hamburg (DE); Kröpke, Rainer, 22869 Schenefeld (DE); Schulz, Jens, Dr., 22869 Schenefeld (DE); Von der Fecht, Stephanie, 22880 Hamburg (DE)
(74) Vertreter: Hartmann, Jost

(56) Entgegenhaltungen:
- EP-A- 1 627 668
- DE-A1- 3 621 686
- FR-A- 2 879 499
- US-A- 5 073 573
- US-A- 5 882 657
- US-A1- 2004 258 628
- US-B1- 6 428 777
- US-B1- 6 592 883
- GOTTSCHALK: "International Cosmetic Ingredient Dictionary and Handbook 11th Ed. Vol. 4" 2006, THE COSMETIC, TOILETRY AND FRAGRANCE ASSOCIATION , WASHINGTON , XP002521056 * Seite 4503 *

## Beschreibung

Die Erfindung umfasst kosmetische Zubereitungen auf Emulsionsbasis mit einem geringen Emulgatoranteil umfassend naphtenarme bzw. -freie Mineralöle und quaternierte, bevorzugt tonmineralische Stabilisatoren.
Die Zubereitungen zeichnen sich durch eine gute Stabilität und verminderter Ölabscheidung aus, wodurch eine nicht klebrige Sensorik der Zubereitungen erzielt wird.

Aus dem Stand der Technik sind eine Vielzahl emulsionsbasierter kosmetischer Zubereitungen bekannt, die zur Verbesserung der Verteilbarkeit der Zubereitung auf der Haut Mineralöle enthalten.

Öle sind wohl die ältesten Produkte bzw. Produktbestandteile von Hautpflegezubereitungen. Meist werden dabei Mischungen verschiedener Fettsäureester, flüssiger Fettalkohole, Wollwachs-Derivate und nicht trocknende Pflanzenöle wie Mandelöl, Weizenkeim- oder Avocadoöl eingesetzt.
Seit Beginn des Erdölzeitalters stehen zusätzlich Mineralöle, Vaseline und andere Paraffine als bei Raumtemperatur flüssige, halbfeste oder feste Ölkomponenten, auch allgemein als lipophile Verbindungen bezeichnet, für die Herstellung von kosmetischen Formulierungen zur Verfügung. Diese Erdölprodukte haben den Vorteil weniger oxidationsempfindlich zu sein als pflanzliche Öle. Da sie in unterschiedlicher Viskosität (fest, cremig, flüssig) erhältlich sind, kann mit ihnen die Konsistenz der kosmetischen Zubereitung modelliert werden. Nicht zuletzt sind sie in der Regel deutlich kostengünstiger als pflanzliche Öle.

Erdölprodukte wie Vaseline, Mikrowachse, dünnflüssige Paraffine, Mineralöle oder Tafelparaffine sind "Naturprodukte", die aus einem destillativen Reinigungsprozess gewonnen werden.

Mineralöl stellt eine Sammelbezeichnung für die aus mineralischen Rohstoffen (Erdöl, Braun- und Steinkohlen etc.) gewonnenen flüssigen Destillationsprodukten dar. In Kosmetika und pharmzeutischen Produkten werden heute - und teilweise schon seit über 100 Jahren - ausschließlich vollhydrierte, hochgereinigte Qualitäten, die so genannten pharmazeutischen Weißöle nach DAB eingesetzt. Sie sind geruch- und farblos, sowie besonders stabil. Anzumerken ist ferner, dass sehr ähnliche Substanzmischungen, die sogenannten Mineralwachse, natürlicherweise in relativ hohen Mengen auch in verschiedenen Pflanzenwachsen (z.B. Candellilawachs) und Insektenwachsen (z.B. Bienenwachs) enthalten sind. Es handelt sich bei diesen Verbindungen um Stoffgemische, die je nach Herkunft unterschiedlich zusammengesetzt sind. Beispielsweise sind Produkte, die aus geologisch altem venezuelanischem Erdöl gewonnen wurden besonders reich an Naphthenen (Cycloalkanen). Das geologisch junge Nordseeöl hingegen ist arm an Naphthenen und enthält überwiegend acyclische Verbindungen.
Naphtenreiche Mineralöle gibt es nur in ausgewählten Gebieten dieser Welt (Venezuela, Saudi-Arabien, Russiand). Sie sind schwierig zu gewinnen und dementsprechend teuer. Naphtenarme Mineralöle sind leichter zu gewinnen und eher als preiswert einzustufen. Nachteil der napthenarmen Mineralöle ist, dass diese Öle in Emulsionen eingesetzt die Emulsionen destabilisieren, was eine starke Ölabscheidung zur Folge hat.

Das zeigen auch folgende Untersuchungsergebnisse.
Es wurde eine kohlenwasserstoffhaltige Wasser-in-Öl Creme mit 4 kommerziell erhältlichen Mineralölen unterschiedlicher Zusammensetzung, bei ansonsten identischer Zusammensetzung der Cremes, hergestellt und die FestigkeitlKonsistenz und die Neigung zur Ausölung bestimmt.

Die 4 Mineralöle weisen die folgende Zusammensetzung auf (bestimmt nach DIN 51378) in Gewichtsprozent, bezogen auf die Gesamtmasse der Öle:

**Tabelle 1: Zusammensetzung Mineralöle**

| | Mineralöl 1 | Mineralöl 2 | Mineralöl 3 | Mineralöl 4 |
|---|---|---|---|---|
| Cycloalkane (Naphthene) | 46-51% | 38-46% | 34-38% | - |
| Paraffinketten | 52-57% | 54-62% | 62-66% | 100% |
| Aromaten | - | - | - | - |
| Klassifizierung | naphthenreich | naphthenisch | naphthenarm | naphthenfrei |

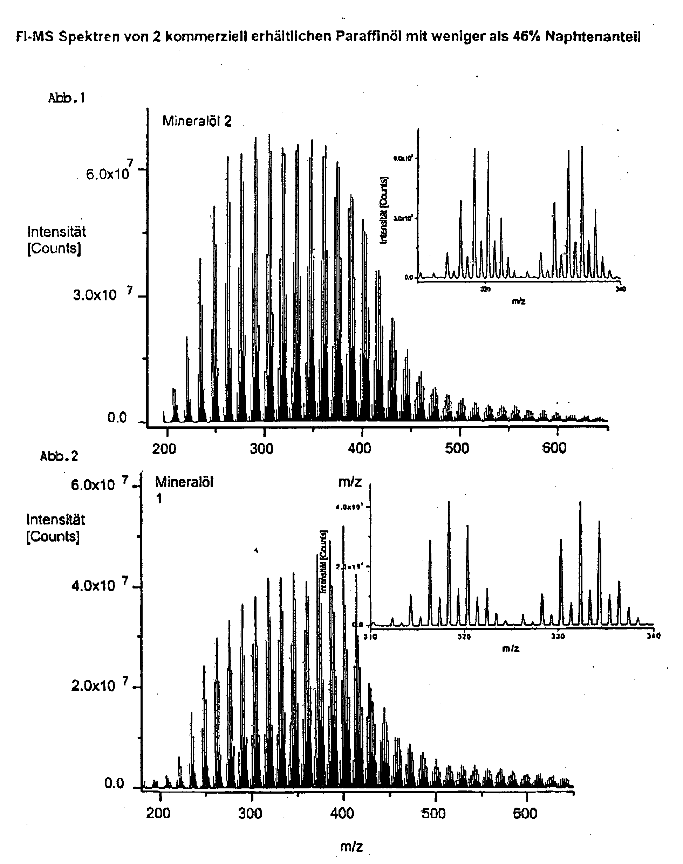

Die in Abbildung 1 und 2 dargestellten Massenspektren zeigen die unterschiedliche Zusammensetzung zweier kommerziell erhältlicher Paraffinöl mit weniger als 50 % bzw. weiniger als 46% Naphtenanteil (Mineralöl 1 und Mineralöl 2).

Mittels Feldionisations-Massenspektrometrie (FI-MS) kann dabei nicht nur die verschiedenen Massenbereiche abgebildet werden, sondern auch die Naphthenverteilung untersucht werden (siehe Ausschnittsvergrößerungen in den Abbildungen). Je nach Anzahl der vorhandenen Ringe pro Molekül variieren die Molmassen der Kohlenwasserstoffe.

Naphtene oder alizyklische Kohlenwasserstoffe sind ringförmige Kohlenwasserstoffe. Der Naphtengehalt vom Rohöl liegt in der Regel bei 5 %, beim russischen Öl liegt er häufig darüber, bei amerikanischem darunter. Naphtene haben in der Molekularstruktur eine höhere Bindungsspannung als Paraffine und besitzen daher einen höheren Heizwert.

Cycloalkane (Cycloparaffine) sind gesättigte ringförmige Kohlenwasserstoffe der allgemeinen Formel CₙH₂ₙ ( n = 3,4,5.....), deren Namen aus dem des entsprechenden Alkanes und der Vorsilbe Cyclo- gebildet werden. Die im Erdöl vorkommenden Cycloalkane u.a. Cyclopentan und Cyclohexan werden auch Naphtene genannt.

Naphtenfreie Mineralöle enthalten nur lineare Kohlenwasserstoffe (CₙHₘ) und weisen daher andere Massenpeaks im FI-MS-Spektrum auf (Mineralöl 4).

### Bestimmung der Konsistenz der Zubereitungen:

Es wurde mit einem Konsistenzbestimmungsgerät nach Fligge (gem. DE 29 09 087) die Konsistenz der Creme-Zubereitungen umfassend die Mineralöle 1 - 4 nach unterschiedlichen Zeitabständen gemessen. Beispielsweise zeigte sich nach 7 Tagen die folgende Konsistenz/Festigkeit der Cremes:

**Tabelle 2: Konsistenz der Cremes mit Mineralöl unterschiedlichen Naphtenanteils**

| Creme mit | Mineralöl 1 | Mineralöl 2 | Mineralöl 3 | Mineralöl 4 |
|---|---|---|---|---|
| Konsistenz nach 7 Tagen | 46 Skt | 44 Skt | 40 Skt | 19 Skt |

| | | | | |
|---|---|---|---|---|
| Skt = Skalenteile | | | | |

Je geringer der Wert der Skalenteile, desto weicher ist die Creme.

### Bestimmung der Tendenz zur Ausölung:

Bei einer Lagertemperatur von 40 °C wurde über einen Zeitraum von 10 Stunden ein 135 mm langer Papierstreifen in die jeweiligen Cremes eingetaucht und anschließend die Höhe gemessen, bis zu welcher das Mineralöl gelaufen ist. Eine lange Laufstrecke (große Steighöhe) steht dabei für eine hohe Neigung zur Ausölung. Ausölung bedeutet eine Phasentrennung, bei der sich das Öl aus der Zubereitung abscheidet.
Zubereitungen unterschiedlichen Alters wurden vermessen. Exemplarisch sind nachfolgend in Tabelle 3 die Ergebnisse einer 7 Tage alten Creme angegeben.

**Tabelle 3: Ausölung der Cremes mit Mineralöl unterschiedlichen Naphtenanteils**

| Creme mit | Mineralöl 1 | Mineralöl 2 | Mineralöl 3 | Mineralöl 4 |
|---|---|---|---|---|
| Steighöhe des Öls in mm | 65 mm | 72 mm | 94 mm | 135 mm |

Die Untersuchungen bestätigen, dass je höher der Anteil an naphthenischen Komponenten im Mineralöl ist, desto viskoser und stabiler wird die Creme. Die Gefahr des Ausölens sinkt mit zunehmenden Naphthengehalt.

Aufgrund der hohen Kosten für napthenreiche Mineralöle besteht aber der Wunsch auch napthenarme oder napthenfreie Mineralöle für die Herstellung emulsionsbasierter Zubereitungen zu verwenden, obwohl wie dargelegt, diese Öle Nachteile aufweisen.

Wünschenswert ist es daher eine kosmetische Zubereitung auf Emulsionsbasis zur Verfügung zu stellen, die einerseits günstig herstellbar ist und die sich andererseits aber auch durch eine verminderte Ölabscheidung auszeichnet.
Wünschenswert ist es dabei auch den Emulgatoranteil der Zubereitung zu vermindern, um eine vorteilhafte, hautpflegende und stabile Zubereitung zu erhalten.

Als Stabilisatoren kosmetischer Zubereitungen sind neben diversen chemischen Verbindungen auch Bentonite bekannt.
Bentonite ist eine Bezeichnung für Tone und Gesteine, die Smektite, v.a. Montmorillonit, als Hauptminerale enthalten, Daneben können Glimmer, Illit, Cristobalit und Zeolithe als Verunreinigungen in Bentoniten vorhanden sein.
Besonders gereinigte Bentonite vom Natrium-Bentonite-Typ gelangen als Stabilisatoren, Dispergiermittel oder Verdickungsmittel in den Handel. Durch Zugabe von Methylcellulose (0,02%) bzw. von Natrium-Carboxymethylcellulose (0,02-1%) können Bentonite-Suspensionen stabilisiert werden. Beim Einsatz von Bentoniten muß allerdings berücksichtigt werden, dass Bentonite Wirkstoffe zu adsorbieren und damit möglicherweise zu inaktivieren vermögen.

Die DE 10321147 beschreibt emulsionsbasierte kosmetische Zubereitungen mit selbstbräunenden Substanzen umfassend Bentonite, die zur Stabilisierung der selbstbräunende Substanzen, wie z.B. DHA, dienen. DHA zersetzt sich und zerfällt in saure Bestandteile, wodurch diese Emulsionen besonders stabilisiert werden müssen.

US 5 073 573 A (MARTIN ROLAND [DE] ET AL) 17. Dezember 1991 (1991-12-17) offenbart kosmetische Zusammensetzungen auf Basis von Schichtsilikat + Mineralöl (Spalte 2, Zeile 16; auch Tabelle 12 und 14). Die Stabilität ölhaltiger Zusammensetzungen wurde untersucht: die verminderte Ölabscheidung wird auf Al Mg Hydroxypalmitat zurückgeführt (Spalte 24, Zeile 9).

Wünschenswert ist es daher auch eine kosmetischen Zubereitungen mit verbesserter Stabilität zur Verfügung zu stellen.

Die Anmeldung offenbart kosmetische Zubereitungen auf Emulsionsbasis mit einem geringem Emulgatoranteil und napthenarmen und/oder napthenfreien Mineralölen sowie quaternierte Stabilisatoren, bevorzugt quaternierte Bentonite.

Napthenarme Mineralöle werden erfindungsgemäß definiert als Mineralöle mit einem Napthenanteil gleich oder unterhalb 46 Gew.% (bestimmt nach DIN 51378), bezogen auf die Gesamtmasse des Öls.
Bevorzugt umfassen die Mineralöle einen Napthenanteil von gleich oder weniger als 38 Gew.%, insbesondere sind sie napthenfrei (0 Gew.%).

Bevorzugt zu verwendende Mineralöle sind die im Handel unter der Bezeichnung Ondina 917, Ondina 927 und/oder Ondina 919 der Fa. Shell, Pionier 6301 S, Pionier 2076 und/oder Pionier 1148 der Fa. Hansen & Rosenthal und/oder Paraffin Oil BF3 0025 von der Fa. RAM Oil.
Diese naphtenarmen Öle sind farblose, geruch- und geschmackneutrale, pharmazeutische Weissöle, die den Spezifikationen US FDA 21 CFR 178.3620 (a), US FDA 21 CFR 172.878. US FDA "White Mineral Oil" und Europ. Pharmacopeia III Edition gehorchen.

Als quaternierte Stabilisatoren können bevorzugt ein oder mehrere Rohstoffe gewählt werden aus der Gruppe Quaternium-18 Hektorit, Quaternium-90 Bentonite und/oder Quaternium-18 Bentonite.
Der Begriff quaterni bedeutet je vier, vierfach, zu viert, abgeleitete Bezeichnung für Reaktionen, bei denen geeignete Atome in einer organischen Verbindung durch vollständige Alkylierung in vierfach substituierte Derivate überführt werden.

Bevorzugt werden quaternierte Bentonite, auch organophile Bentonite, als erfindungsgemäße quaternierte Stabilisitatoren eingesetzt. Vorteilhafte modifizierte Schichtsilikate im Sinne der vorliegenden Erfindung sind beispielsweise durch Umsetzung von Schichtsilikaten mit Quaternium-18 erhältlich. Quaternium-18 ist eine Mischung von quaternären Ammoniumchloridsalzen, welche durch die folgende Strukturformel beschrieben werden: 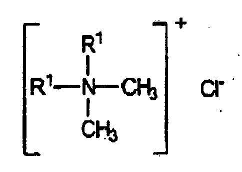 worin die Reste R¹ unabhängig voneinander gewählt werden aus hydrierten Talgresten mit einer Kettenlänge von 12 bis 20 Kohlenstoffatomen.
Erfindungsgemäß besonders bevorzugt sind Stearalkoniumhektorit, ein Reaktionsprodukt aus Hektorit und Stearalkoniumchlorid (Benzyldimethylstearylammoniumchlorid), und Quaternium-18 Hektorit, ein Reaktionsprodukt aus Hektorit und Quaternium-18, welche z. B. unter den Handelsbezeichnungen Bentone 27 und Bentone 38 bei Nordmann & Rassmann erhältlich sind.
Ganz besonders bevorzugt ist erfindungsgemäß Quaternium-90 Bentonite, ein Reaktionsprodukt aus Bentonite und Quaternium-90, das von der Firma Süd-Chemie unter dem Handelsnamen Tixogel VP-V zu beziehen ist. Die Bezeichnung deutet daraufhin, dass die Alkylreste R¹ bei diesem Produkt pflanzlichen Ursprungs sind, wodurch sich besonders vorteilhafte Eigenschaften im Sinne der vorliegenden Erfindung bzgl. der Stabilisierung ergeben.
So ist die Kettenlängenverteilung der Alkylreste R¹ typisch für Rohstoffquellen wie hydriertes Palmöl. Der Anteil der Alkylreste R¹ ist wie folgt, wobei sich rohstoffbedingte Schwankungen aufgrund der natürlichen Herkunft ergeben:
C₁₈H₃₁ ca. 55-65 Gew.%. C₁₆H₃₃ ca. 30-40 Gew.%, C₁₄H₂₉ < 5 Gew.%

Ebenso sind hydrophile oder organophile Schichtsilikate, wie beispielsweise synthetischer oder natürlicher Hectorit, erhältlich von der Firma Süd-Chemie unter dem Namen Otpigel® oder Tixogel®, besonders als quaternierte Stabilisatoren bevorzugt.

Als quarternierte Stabilisatoren werden ein oder mehrer Verbindungen aus der Gruppe der synthetischen, natürlichen, hydrophilen und/oder organophilen Schichtsilikate, insbesondere Betonite und/oder Hectorite, bevorzugt Quaternium-90 Bentonite, Quaternium-18 Bentonite, Quaternium-18 Hectorite, gewählt..

Die Gesamtmenge an einem oder mehreren quaternierten Stabilisatoren in der fertigen Zubereitung wird vorteilhaft aus dem Bereich von 0,05 bis 10,0 Gew.%, bevorzugt von 0,1 bis 5 Gew.%, besonders bevorzugt von 1.0 bis 3.0 Gew.°/o gewählt, bezogen auf das Gesamtgewicht der Zubereitungen.

Überraschenderweise konnte mit Hilfe der erfindungsgemäßen quaternierten Stabilisatoren napthenarme bzw. - freie Mineralöle in kosmetische Zubereitungen auf Emulsionbasis mit geringem Emulgatoranteil kombiniert werden ohne das eine Ölabscheidung zu beobachten ist.
So wurde entsprechend den zuvor ausgeführten Tests zur Ausölung bei erfindungsgemäßen Zubereitungen (z.B. Beispiel 3) eine Laufstrecke von weniger als 70 mm gemessen.

Insbesondere sind die Zubereitungen stabil und eine Ausölung bei hohen Lagertemperaturen wird vermieden.
Verbindungen aus der Gruppe der synthetischen, natüdichen, hydrophilen und/oder organophilen Schichtsilikate, insbesondere Betonite und/oder Hectorite, bevorzugt Quaternium-90 Bentonite, Quaternium-18 Bentonite. Quaternium-18 Hectorite, lassen sich demnach zur Verminderung der Ausölung ölhaltiger kosmetischer Zubereitungen verwenden. Insbesondere lässt sich so der Einsatz von Mineralölen mit niedrigem Naphtengehalt in kosmetischen Zubereitungen ohne Nachteile bewerkstelligen.

Weiterer Vorteil ist, dass mittels der Kombination quarternierter Stabilisatoren und napthenarmen Mineralölen eine angenehme Sensorik der Zubereitungen erhalten wird. Die erfindungsgemäßen Zubereitungen weisen keine ansonsten erwartete unangenehme Klebrigkeit auf.
Die Verwendung von ein oder mehrer Verbindungen aus der Gruppe der synthetischen, natürlichen, hydrophilen und/oder organophilen Schichtsilikate, insbesondere Betonite und/oder Hectorite, bevorzugt Quaternium-90 Bentonite, Quatemlum-18 Bentonite, Quaternium-18 Hectorite zur Verminderung der Klebrigkeit von kosmetischen Zubereitung ist damit in gleicher Weise indiziert.

Insbesondere die Kombination von Quaternium-90 Bentonit mit napthenarmen oder napthenfreien Mineralölen bildet in einer W/O-Emulsion bzw. W/Si-Emulsion oder Si/W-Emulsion mit einem Emulgatoranteil von weniger 2,5% eine kosmetische Zubereitung, die eine Langzeitstabilität aufweist und aufgrund des geringen Emulgatoranteils als wenig klebrig empfunden wird.
Erfindungsgemäß ist ein geringer Emulgatoranteil als weniger 5 Gew.% an Emulgatoren defineirt, insbesondere weniger als 2,5 Gew.%, bezogen auf die Gesamtmasse der Zubereitung. Vorteilhaft ist die erfindungsgemäße Zubereitung emulgatortrei.

Die erfindungsgemäßen Zubereitungen können vorteilhaft weitere kosmetische und/oder dermatologische Wirk-, Hilfs- und Zusatzstoffe enthalten.
Die erfindungsgemäßen kosmetischen Zubereitungen können kosmetische Hilfsstoffe enthalten, wie sie üblicherweise in solchen Zubereitungen verwendet werden, z. B. Antioxidantien, Konservierungsmittel, Parfüme, antimikrobielle Wirkstoffe, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Als Wirkstoffe sind Hautbefeuter (Moisturizer), UV-Filtersubstanzen oder Antioxidantien bevorzugt.
Weitere vorteilhafte Wirkstoffe im Sinne der vorliegenden Erfindung sind natürliche Wirkstoffe und/oder deren Derivate, wie z, B. alpha-Liponsäure, Phytoen, D-Biotin, Coenzym Q10. Folsäure und/oder deren Derivate, alpha-Glucosylrutin, Carnitin, Carnosin, natürliche und/oder synthetische Isoflavonoide, Kreatin, Taurin und/oder β-Alanin.

Erfindungsgemäße kosmetische Zubereitungen können in verschiedenen Formen vorliegen. So können sie z.B. eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), eine W/Si-Emulsion, ein Gel, einen festen Stift, eine Salbe oder auch ein Aerosol auf Emuslionsbasis darstellen.

Bevorzugt ist die erfindungsgemäße Zubereitung auf Basis einer WIO-, W/Si-Ernulsion oder Si/W-Emulsion formuliert, insbesondere als W/O-Emulsion.

Die in den nachfolgenden Beispielen angegebenen Anteilsangaben beziehen sich auf die jeweiligen Gewichtsanteile bezogen auf die Gesamtmasse der Zubereitung.

### Beispiele

**W/O-Emulsionen**

| | **1** | **2** | **3** | **4** | **5** |
|---|---|---|---|---|---|
| Triglycerindiisostearat | 1,0 | 0,5 | 0,25 | 2,0 | 3,0 |
| Diglycerindipolyhydroxystearat | 1,0 | 1,5 | 1,75 | 3,0 | 2.0 |
| Paraffinöl mit weniger als 46% Naphtenanteil | 12,5 | 10,0 | 8,0 | 5,0 | 17,5 |
| Vaseline | 8.0 | 6.0 | 5,0 | 12.0 | 2,5 |
| hydrierte Kokosglyceride | 2,0 | 1,0 | 2,5 | 5,0 | 0,25 |
| Decyloleat | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0.3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Quaternium-90 Bentonit | 0,5 | 1,0 | 0,75 | 0,25 | 0,1 |
| Magnesiumsulfat | 0,5 | 0,6 | 0,5 | 0,7 | 1,0 |
| Glycerin | 3,0 | 5.0 | 10,0 | 15.0 | 1,5 |
| Zitronensäure | 0,2 | 0,1 | 0,2 | 0,3 | 1,0 |
| Natriumcitrat | 0,2 | 0.05 | 0.4 | 0.3 | 2,0 |
| Parfum | q,s, | q,s, | q,s, | q,s, | q,s, |
| Ethanol | 2,0 | --- | 5,0 | --- | --- |
| Capryl-/Caprinsäuretriglycerid | 2.0 | 2,5 | 3,0 | 5,0 | 0,5 |
| Kaliumsorbat | 0,04 | 0,15 | 0,05 | 0,03 | .J 0,4 |
| Benzylalkohol | 0,3 | 0,4 | 0,25 | 0,15 | --- |
| Talkum | --- | --- | 0,5 | 1,0 | 0,1 |
| Wasser | ad 100 | ad 100 | ad 100 | ad 100 | ad 100 |

**W/O-Emulsionen**

| | **6** | **7** | **8** | **9** | **10** |
|---|---|---|---|---|---|
| PEG-30 Dipolyhydroxystearat | --- | 0,5 | 0,25 | --- | 3,0 |
| Lanolin Alcohol | 1,0 | 1,5 | 1,75 | 3,0 | -- |
| Paraffinöl mit weniger als 46% Naphtenanteil | 12,5 | 10,0 | 8,0 | 5,0 | 17,5 |
| Vaseline | 8,0 | 6,0 | 5,0 | 12,0 | 2.5 |
| hydrierte Kokosglyceride | 2,0 | 1,0 | 2,5 | 5,0 | 0,25 |
| Hydriertes Polyisobuten | 0,5 | 0,75 | 1,0 | 2,0 | 0,25 |
| Octyldodecanol | 0,5 | 1,0 | 0,75 | 13,0 | 0,25 |
| Alurniniumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2.5 | 5,0 |
| Quaternium-90 Bentonit | 0,5 | 1,0 | 0,75 | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Parfum | q,s, | q,s, | q,s, | q,s, | q,s, |
| 1,3 Butylenglykol | 2,0 | --- | 5,0 | --- | --- |
| Capryl-/Caprinsäuretriglycerid | 2,0 | 2,5 | 3,0 | 5,0 | 0,5 |
| Natriumdehydracet | --- | --- | 0,05 | --- | --- |
| Kaliumsorbat | 0,3 | 0,4 | 0,25 | 0,15 | 0,05 |
| Talkum | --- | --- | 0,05 | --- | 0,1 |
| Wasser | ad 100 | ad 100 | ad 100 | ad 100 | ad 100 |

**W/Si-Emulsion**

| | **11** | **12** | **13** | **14** | **15** |
|---|---|---|---|---|---|
| Cetyl PEG/PPG-10/1 Dimethicone | 1,0 | --- | -- | 3,0 | 5,0 |
| Cylomethicon + PEG/PPG-18/18 Dimethicon (90:10) | 7,5 | 5,5 | 5 | -- | -- |
| Paraffinöl mit weniger als 46% Naphtenanteil | 2,5 | 1,0 | 8,0 | 5,0 | 7,5 |
| Cyclomethicon | 12,5 | 15 | 28,0 | 25,0 | 17,5 |
| Dimethicon | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Natriumcitrat | 1,0 | 0,1 | 0,4 | 0,9 | 2,5 |
| Parfum | q,s, | q,s. | q,s, | q,s, | q,s, |
| Kaliumsorbat | 0,4 | 0,1 | 0,05 | 0,3 | 0,4 |
| Quaternium-90 Bentonit | 1,0 | 0,1 | 0,5 | 0,25 | 0,1 |
| Cetyldimethicon | 0,5 | -- | 0,7 | --- | --- |
| Benzylalkohol | --- | --- | 0,05 | --- | 0,1 |
| modifizierte Stärke | --- | 2,5 | --- | 0,15 | -- |
| Wasser | ad 100 | ad 100 | ad 100 | ad 100 | ad 100 |

**W/Si-Emulsionen**

| | **16** | **17** | **18** | **19** | **20** |
|---|---|---|---|---|---|
| Cetyl PEG/PPG-10/1 Dimethicone | 1,0 | --- | -- | 3,0 | 5,0 |
| Cylomethicon + PEG/PPG-18/18 Dimethicon (90:10) | 6,0 | 7,5 | 5 | -- | -- |
| Cyclomethicon | 12,5 | 15 | 8,0 | 25,0 | 17,5 |
| Paraffinöl mit weniger als 46% Naphtenanteil | 5,5 | 3,0 | 8,0 | 5,0 | 7,5 |
| Dimethicon | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2.0 | 0,6 | 2,5 | 0,7 | 1,0 |
| Glycerin | 3,0 | 5,0 | 10,0 | 15,0 | 1,5 |
| Milchsäure | 0,2 | 0,1 | 0,2 | --- | --- |
| Natriumlactat | 0,2 | 1,0 | 0.05 | --- | --- |
| Parfum | q,s. | q,s, | q,s, | q,s, | q,s, |
| Talkum | 1,0 | --- | --- | --- | 0,5 |
| Quaternium-90 Bentonit | 0,25 | 0,1 | 2,5 | 1,5 | 1,1 |
| Kaliumsorbat | 0,3 | 0,4 | 0,25 | 0,15 | 0,05 |
| Stearyldimethicon | 0,5 | --- | 0,7 | --- | --- |
| Dehydracetsäure | --- | --- | 0,05 | --- | 0,1 |
| modifizierte Stärke | --- | 2,5 | --- | 0,15 | --- |
| Wasser | ad 100 | ad 100 | ad 100 | ad 100 | ad 100 |

**W/O-Emulsionen**

| | **21** | **22** | **23** | **24** | **25** |
|---|---|---|---|---|---|
| PEG-22 Dodecyl Glycol Copolymer | 4,0 | 1,5 | 0,25 | --- | 3,0 |
| PEG-45 Dodecyl Glycol Polymer | 1,0 | 1,5 | 1,75 | 3,0 | -- |
| Paraffinöl mit weniger als 46% Naphtenanteil | 12,5 | 10,0 | 8,0 | 5,0 | 17,5 |
| Isopropylstearat | 8,0 | 6,0 | 5,0 | 12,0 | 2,5 |
| hydrierte Kokosglyceride | 2,0 | 1,0 | 2,5 | 5.0 | 0,25 |
| Nachtkerzenöl | 0,5 | 0,75 | 1,0 | 2,0 | 0,25 |
| Octyldodecanol | 0,5 | 1,0 | 0,75 | 3,0 | 0,25 |
| Dicaprylylcarbonat | 0,1 | 0,05 | 0,15 | 0,5 | 1,0 |
| Quatemium-90 Bentonit | 0,5 | 1,0 | 0,75 | 0,25 | 5,0 |
| Magnesiumsulfat | 0,5 | 0,6 | 0,5 | 0,7 | 1,0 |
| Glycerin | 3.0 | 5,0 | 10,0 | 15,0 | 1,5 |
| Natriumcitrat | 0,2 | 0,1 | --- | --- | --- |
| Zitronensäure | 0,2 | 0,1 | --- | --- | --- |
| Parfum | q.s, | q,s, | q,s, | q,s, | q,s, |
| 1,3 Butylenglykol | 2.0 | --- | 5,0 | --- | --- |
| Capryl-/Caprinsäuretriglycerid | 2,0 | 2,5 | 3,0 | 5,0 | 0,5 |
| Kaliumsorbat | 0,4 | 0,15 | 0,05 | 0,3 | 0,4 |
| Talkum | 0.3 | 0,4 | 0,25 | 0,15 | --- |
| Benzylalkohol | --- | --- | 0,05 | --- | 0,1 |
| Wasser | ad 100 | ad 100 | ad 100 | ad 100 | 1 ad 100 |

**W/O-Emulsionen**

| | **26** | **27** | **28** | **29** | **30** |
|---|---|---|---|---|---|
| Polyglyceryl-2-dipolyhydroxystearat | 3,0 | --- | 0,25 | --- | 3,0 |
| Polyglyceryl-3 diisostearate | 1,0 | 3,5 | 1,75 | 1,5 | -- |
| PEG-40 sorbitanisostearate | --- | 1,5 | 0,5 | 3,5 | 2,0 |
| Paraffinöl mit weniger als 46% Naphtenanteil | 12,5 | 10,0 | 8,0 | 5,0 | 17,5 |
| Isopropylstearat | 8,0 | 6,0 | 5,0 | 12,0 | 2,5 |
| hydrierte Kokosglyceride | 2,0 | 1,0 | 2.5 | 5,0 | 0,25 |
| Isopropylpalmitat | 0,5 | 1,0 | 0,75 | 3,0 | 0,25 |
| Dicaprylylcarbonat | 0,1 | 0,05 | 0,15 | 0,5 | 1,0 |
| Quatemium-90 Bentonit | 0,5 | 1,0 | 0,75 | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0.2 | 0,1 | 0,1 | 0,3 | 1,0 |
| Natriumcitrat | 0,2 | 0,3 | 0,2 | 1,5 | 0.8 |
| Parfum | q,s, | q,s, | q,s, | q,s, | q,s, |
| Capryl-/Caprinsäuretriglycerid | 2.0 | 2,5 | 3,0 | 5,0 | 0,5 |
| Kaliumsorbat | 0,24 | 0.15 | 0,05 | 0,3 | 0,4 |
| Dehydracetsäure | --- | --- | --- | --- | 0,05 |
| Benzylalkohol | --- | --- | --- | --- | 0,05 |
| Talkum | --- | --- | --- | --- | 0,5 |
| Wasser | ad 100 | ad 100 | ad 100 | ad 100 | ad 100 |

## Patentansprüche

1. Verwendung von ein oder mehreren Verbindungen aus der Gruppe der synthetischen, natürliche, hydrophilen und/oder organophilen Schichtsilikate, zur Verminderung der Ausolung ölhaltiger kosmetischer Zubereitungen umfassend ein oder mehrere Mineralöle mit einem Naphthenanteil gleich oder unterhalb 46 Gew.%, bezogen auf die Gesamtmasse des Mineralöl.

2. Verwendung nach Anspruch 1, **dadurch gekennzeichnet, dass** das oder die Mineralöle naphtenfrei sind.

3. Verwendung nach Anspruch 1 oder 2, **dadurch gekennzeichnet, dass** als Stabilisatoren ein oder mehrer Verbindungen aus der Gruppe der synthetischen, natürlichen, hydrophilen und/oder organophilen Schichtsilikate, insbesondere Betonite und/oder Hectorite, bevorzugt Quaternium-90 Bentonite, Quaternium-18 Bentonite, Quaternium-18 Hectorite, gewählt werden.

4. Verwendung nach einem der vorhergehenden Ansprüche, **dadurch gekennzeichnet, dass** sie einen Emulgatoranteil von weniger als 2,5 Gew.% enthalten, bezogen auf das Gesamtgewicht der Zubereitungen.

5. Verwendung nach einem der vorhergehenden Ansprüche, **dadurch gekennzeichnet, dass** sie emulgatorfrei sind.

6. Verwendung nach einem der vorhergehenden Ansprüche, **dadurch gekennzeichnet, dass** sie als W/O-Emulsion, W/Si-Emulsion oder SI/W-Emulsion vorliegen, bevorzugt als W/O-Emulsion.

## Claims

1. Use of one or more compounds from the group of synthetic, natural, hydrophilic and/or organophilic sheet silicates for reducing the oiling-out of oil-containing cosmetic preparations comprising one or more mineral oils with a naphthene fraction equal to or less than 46% by weight, based on the total mass of the mineral oil.

2. Use according to Claim 1, **characterized in that** the mineral oil or oils are naphthene-free.

3. Use according to Claim 1 or 2, **characterized in that** the stabilizers selected are one or more compounds from the group of synthetic, natural, hydrophilic and/or organophilic sheet silicates, in particular bentonites and/or hectorites, preferably quaternium-90 bentonite, quaternium-18 bentonite, quaternium-18 hectorite.

4. Use according to one of the preceding claims, **characterized in that** they comprise an emulsifier fraction of less than 2.5% by weight, based on the total weight of the preparations.

5. Use according to one of the preceding claims, **characterized in that** they are emulsifier-free.

6. Use according to one of the preceding claims, **characterized in that** they are in the form of W/O emulsion, W/Si emulsion or Si/W emulsion, preferably W/C emulsion.

## Revendications

1. Utilisation d'un ou de plusieurs composés du groupe des silicates à couches synthétiques, naturels, hydrophiles et/ou organophiles, pour diminuer le déshuilage de compositions cosmétiques contenant de l'huile, comprenant une ou plusieurs huiles minérales présentant une proportion de naphtène égale ou inférieure à 46% en poids par rapport à la masse totale de l'huile minérale.

2. Utilisation selon la revendication 1, **caractérisée en ce que** la ou les huiles minérales sont exemptes de naphtène.

3. Utilisation selon la revendication 1 ou 2, **caractérisée en ce qu'**on choisit, comme stabilisants, un ou plusieurs composés du groupe des silicates synthétiques, naturels, hydrophiles et/ou organophiles, en particulier la bentonite et/ou l'hectorite, de préférence la Quaternium-90 Bentonite, la Quaternium-18 Bentonite, la Quaternium-18 Hectorite.

4. Utilisation selon l'une quelconque des revendications précédentes, **caractérisée en ce qu'**elles contiennent une proportion d'émulsifiant inférieure à 2,5% en poids, par rapport au poids total des compositions.

5. utilisation selon l'une quelconque des revendications précédentes, **caractérisée en ce qu'**elles sont exemptes d'émulsifiant.

6. Utilisation selon l'une quelconque des revendications précédentes, **caractérisée en ce qu'**elles se trouvent sous forme d'émulsion E/H, d'émulsion E/Si ou d'émulsion Si/E, de préférence sous forme d'émulsion E/H.
